**Europäisches Patentamt**

**European Patent Office**

**Office européen des brevets**

(11) Publication number: **0 187 827**
**B1**

(12) **EUROPEAN PATENT SPECIFICATION**

(45) Date of publication of patent specification: **28.11.90**

(21) Application number: **85903611.3**

(22) Date of filing: **08.07.85**

(86) International application number:
**PCT/US85/01287**

(87) International publication number:
**WO 86/00682 30.01.86 Gazette 86/03**

(51) Int. Cl.⁵: **F 16 K 37/00, F 16 P 1/00, F 16 K 5/10, F 16 K 27/00, A 61 M 5/00**

(54) FLOW CONTROL DEVICE FOR ADMINISTRATION OF INTRAVENOUS FLUIDS.

(30) Priority: **13.07.84 US 630632**

(43) Date of publication of application:
**23.07.86 Bulletin 86/30**

(45) Publication of the grant of the patent:
**28.11.90 Bulletin 90/48**

(84) Designated Contracting States:
**BE FR IT LU NL SE**

(56) References cited:
**DE-A-2 735 955   US-A-3 698 683**
**FR-A-2 468 049   US-A-3 807 691**
**US-A- 961 950    US-A-3 998 227**
**US-A- 994 409    US-A-4 079 737**
**US-A-1 131 480   US-A-4 140 297**
**US-A-2 911 008   US-A-4 183 499**
**US-A-3 276 472   US-A-4 256 132**
**US-A-3 323 774   US-A-4 361 147**
**US-A-3 341 168**

(73) Proprietor: **MASTER MEDICAL CORPORATION.**
**7033 East First Avenue**
**Scottsdale, Arizona 85251 (US)**

(72) Inventor: **ASLANIAN, Jerry, L.**
**4247 E. Hazelwood**
**Phoenix, AZ 85018 (US)**

(74) Representative: **Plucker, Guy et al**
**OFFICE KIRKPATRICK 4 Square de Meeu7s**
**B-1040 Bruxelles (BE)**

Courier Press, Leamington Spa, England.

## Description

The present invention relates to a device for regulating and controlling the flow of intravenous (IV) and parenteral fluids. More particularly, the present invention relates to a flow metering device for precisely establishing and maintaining a preselected flow rate during the administration of IV liquids to the patient.

The gravity administration of fluids by IV infusion is a common medical procedure. Representative intravenous fluids include blood, plasma, dextrose and isotonic saline solutions. IV infusions are generally carried out with the container of IV fluid suspended above the patient. Customarily such containers have a seal which is broken by the insertion of a spike and the fluid is delivered to the patient at an administration needle through a drip chamber and flexible tubing connected to the spike. The purpose of the drip chamber is to facilitate the determination of the flow or drip rate through the tubing. The infusion rate is generally regulated by use of an external pinch valve or roller clamp associated with the tubing for the more common gravity type infusions.

Initially when infusions are carried out, the tubing and needle are purged by air by initiating a flow of fluid through the tubing. The needle is then inserted into a venapuncture site at a suitable location such as in the forearm or wrist of the patient and infusion of fluid is initiated. Preferably when the venapuncture site occurs in the lower arm of the patient, the arm should be properly stabilized on a contoured IV arm support. Medical personnel administering the IV will adjust the pinch valve or roller to restrict the IV tubing and the number of drops passing through the drip chamber are counted. The appropriate flow rate is established by trial and error, by progressively restricting or opening the tubing at the pinch valve.

The administration procedure described above requires the attention of medical personnel for a substantial time in the initial establishment of the proper flow rate and in continual monitoring. It is the general practice of medical personnel to periodically check the flow rate by counting the drops of fluid that pass through the drip chamber. Conventional procedures as described not only require substantial time but are often inaccurate. Temperature changes cause expansion and contraction of the IV tubing allowing the flow rate to vary. The tubing may loose "memory" and collapse under continuous squeezing necessitating constant readjustment of the setting. Roller clamps and pinch valves of the general type described have a tendency to slip off the tubing which can pose a threat to the patient.

Independent tests have indicated that conventional pinch valves and roller clamps maintain flow only within about 25% accuracy thereby requiring constant readjustment. Accordingly, it will be appreciated that control of infusion rates with an acceptable degree of accuracy using conventional pinch valves and roller clamps is extremely difficult even with constant attention on the part of attending medical personnel.

Various expedients have been resorted to in an effort to correct the problem set forth above. Flow regulating devices of various types have been developed and can be found in the prior art. Document US—A—3,785,378 to Stewart shows a valve for the administration of intravenous fluids which has an annular member forming a central passage through which fluid is flowable to an end face having multiple grooves. The inner ends of the multiple grooves communicate with the passage and a flow control member is rotatable to place the flow port successively and selectively in communication with the grooves to vary the flow rate.

Another approach to the problem is found in US—A—3,877,428 to Seagle et al which patent shows an infusion control device for selectively controlling the rate of administration of fluids to a patient. The control device is attachable along the IV tubing and includes a rotatable metering member defining a capillary flow path between the input and output of the control device. A metering plate is axially rotatable with respect to the input and output ports to vary the effective length of the flow path so as to regulate the flow between full flow and zero flow conditions.

A somewhat similar approach is shown in US—A—3,880,401 which discloses a flow metering valve having inner and outer component parts which are movable relative one to another at screw threads to effect relative axial movement of a metering valve plug with respect to a metering bore for regulating and terminating flow through the valve flow passage.

Prior documents US—A—4,294, 246 and 4,361,147, commonly assigned with the present application, disclose devices which represent substantial improvements over the prior art. A flow passage is provided within a housing and is connectable to a source of IV fluid and to a delivery tube terminating at an administration needle. In the metering apparatus, a metering pin is axially movable within the flow passage relative to a valve seat and defining a flow passageway and a variable area flow notch which are positionable relative to the valve seat to regulate flow from a full flow purge position to a flow blocking position. In the preferred embodiment, the positioning of the metering pin is accomplished by a cam engaging a portion of the metering pin which forms a cam follower. The cam is manually adjustable by a dial on the device to accomplish accurate, repeatable and continuous flow adjustment over a full range. The system incorporates the metering apparatus, source of IV fluid, drip chamber and administration means. This apparatus and system works particularly well providing a high degree of accuracy and is competitive even with electronic flow controllers.

Document DE—A—2 735 955 discloses a valve having a flow control member rotatable in a matching housing, in which the fluid flow can be controlled by a passageway of variable cross-sectional area.

The valve according to DE—A—2 735 955 is made of plastic material and is thus suitable for production of disposable medical devices. However, the plastic-to-plastic contact can lead to serious sealing problems or parts made from the same material could stick together, causing troubles, in eveness of adjustments and impeding one-hand control.

Document US—A—4 183 499 discloses a low-pressure, low volume plastic metering valve provided within a housing and a rotatable metering member within this housing. The valve is constructed of two kinds of plastic, with the housing being made of a more flexible and yieldable with memory plastic. The housing can thus accommodate to the more brittle, non yieldable central metering member. The two main pieces being made of plastics of different hardness are able to glide more easily relative to each other, but there remain a problem of durability of the properties of the plastics used and of assembly.

The foregoing devices, particularly those described immediately above, provide substantial improvements over conventional pinch valve and roller clamps, nevertheless are not economically justifiable in some applications or for some medical facilities. Accordingly, there exists a need for a reliable, accurate and repeatable IV flow control device which is simple, effective accurate and which can be provided to the medical care industry at low cost.

Briefly, the present invention achieves the above objects and advantages and provides a unique IV control device which can be adjusted to maintain various settings from zero to full flow with accuracy and repeatability.

The flow control device of the present invention comprises a housing of a first plastic material which has an inlet and outlet port. A flow metering member having a variable area flow passage is rotatable within the housing by means of a manually adjustable dial. The flow metering member selectively places the inlet and outlet port in communication via the flow passageway to regulate the flow from a purge position through a flow adjusting range and to a stop flow position. The housing inlet and outlet are connectable in the IV tubing line which attaches the source of IV fluid to the administration needle. The metering member is fabricated of a second plastic material which is selected to effect sealing and minimize heat shrinkage. In other aspects of the present invention, a housing or backplate member may be selectively attachable to the valving member to provide additional gripping surface for the medical attendant. A security cover may also be associated with the valving member to secure the valving member from tampering or inadvertant adjustment by unauthorized persons.

The present invention relates to a valve for establishing and maintaining predetermined administratioon flow rates in an intravenous system having a source of fluid connectable to an administration needle via tubing, said valve comprising:

(a) a valve housing defining a valve chamber, and including an inlet and an outlet connectable to said tubing and placing said valve chamber in the fluid flow path;

(b) a flow control member rotatable relative to said valve housing;

(c) said valve housing and flow control member defining a flow passageway being at least partially defined by a groove having a first region in which the cross-sectional area varies over its length, and a second flow-blocking region;

(d) means for selectively moving said flow control member relative to said valve housing to interpose a selected portion of said flow passageway in said fluid path to vary the flow rate or block the fluid flow.

In accordance with the preferred embodiment, the valve of the present invention is characterized in that:

(e) said valve chamber is cylindrical and said flow control member includes a barrel having a cylindrical outer surface, said flow passageway being at least partially defined by said groove on this cylindrical surface and having a third region of substantially uniform area for establishing a purge flow condition;

(f) said means for selectively moving said flow control member relative to said valve housing including a separate dial assembly provided with a cylindrical axle and an axial key;

(g) said barrel having a concentric bore and an axial keyway in which are received the axle and the axial key of said dial assembly;

(h) said valve housing and said dial assembly being made of a first sterilizable plastic material which will no flow or deform under hoop stress, the barrel being made of a second plastic material selected to minimize heat shrinkage, the hardness of this second plastic material being lower than the hardness of the first plastic material as represented by Shore hardness to provide sealing and wiping action;

(i) the barrel and the valve housing being engaged in an interference fit with the barrel slightly compressed due to the relative dimensions of the barrel and the valve housing.

Said first plastic material may e.g. be selected from the group consisting of ABS, XT and said second plastic material may be selected from the group consisting of Neoprene, silicone, LDP or PVC.

In a preferred embodiment of the invention, the flow control member is provided with flange means which engage a portion of said valve housing.

Preferably, the valve housing is provided with stop means cooperable with said flow control member to limit movement thereof.

According to a particular embodiment of the invention, the valve further includes a backplate detachably secured to the valve housing. This backplate preferably defines a recess for receiving said valve housing and has a planar body extending from said recess to provide a gripping surface. In accordance with a preferred embodiment of the invention, the valve further includes a cover face plate which is movably securable to said backplate and valve and which has an open position exposing said valve and a closed position covering said valve. In particular, said cover means are securable to said backplate by pivotal means. Preferably, said cover plate is provided with a window therein exposing a part of the dial in said closed position.

The above and other objects and advantages of the present invention will become more readily apparent from the following description, claims and drawings in which:

Figure 1 is a perspective view of a valve assembly including an optional backplate and cover;

Figure 2 is an exploded perspective view of the assembly shown in Figure 1;

Figure 3 is a perspective view similar to Figure 1 with the cover plate removed;

Figure 4 is a sectional view;

Figure 5 is a rear exploded view of the flow control valve and connecting tubing;

Figure 6 is a perspective view similar to Figure 1 with the cover in an open position;

Figure 7 is an exploded view of another embodiment of the metering valve;

Figure 8 is a sectional view of the valve of Figure 7;

Figure 9 is a rear perspective view of an embodiment of the invention; and

Figure 10 is a perspective view of a detachable backplate.

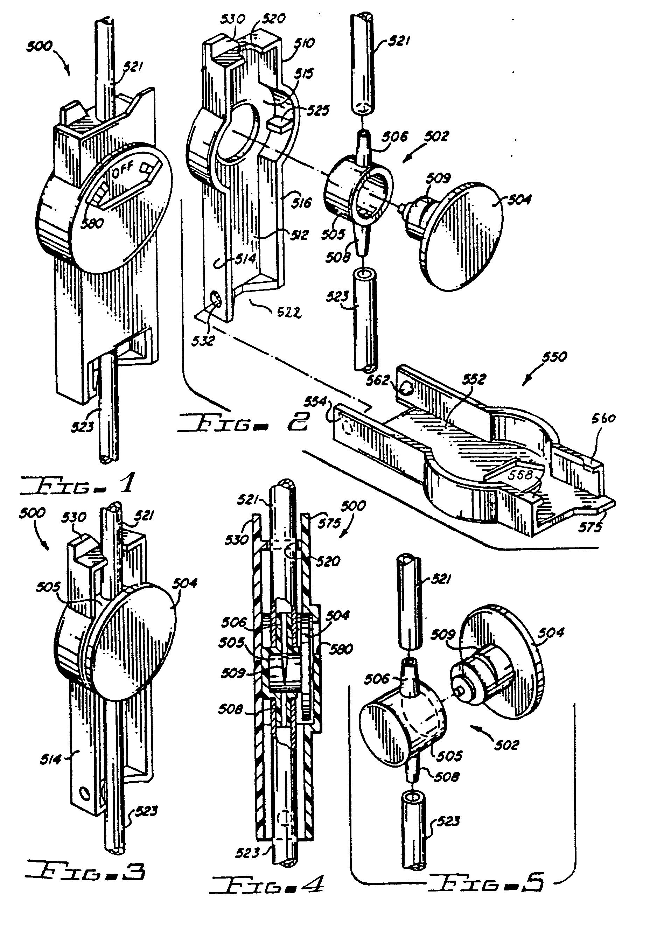

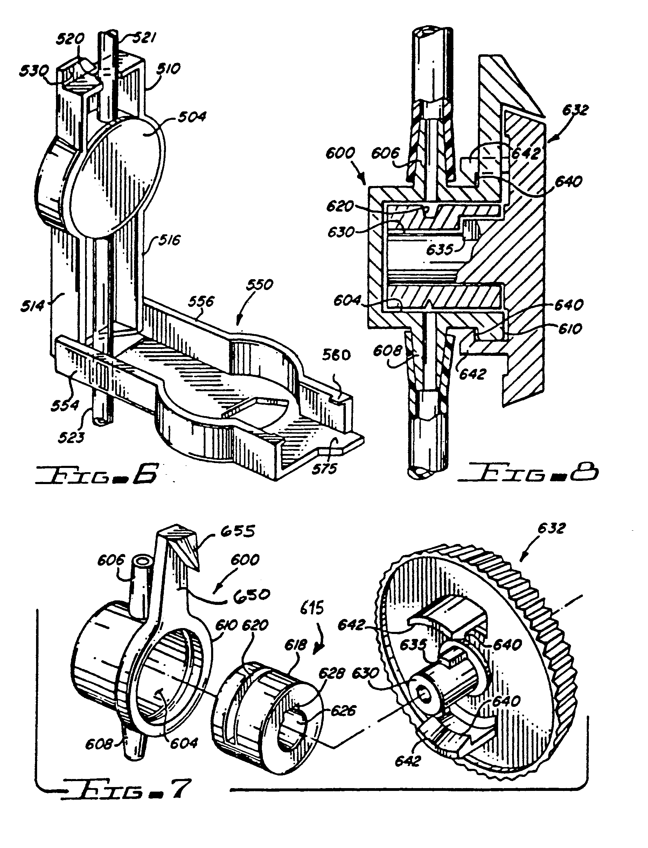

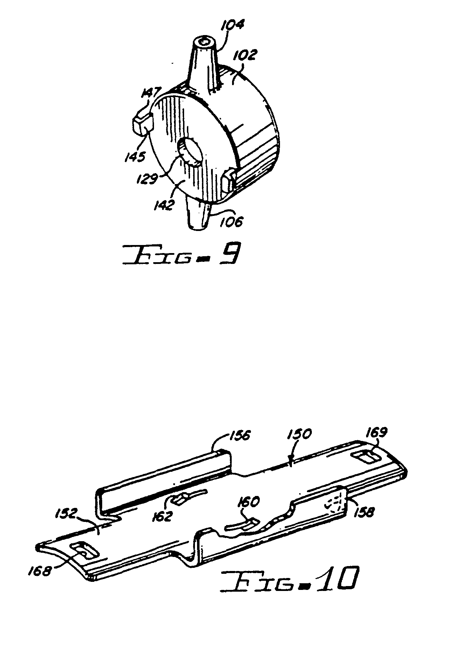

Figure 1 through 6 show embodiment of the present invention which is generally designated by the numeral 500. In this embodiment, the basic flow control device is provided with several accessories which may be included at the option of the user. The embodiment of these figures includes the basic metering valve designated by the numeral 502 which may be in the form of any of the valves heretofore described having a rotary flow control member 504 for adjusting flow through the valve housing 505 and having inlet and outlet fittings 506 and 508 respectively. The basic valve 520 is an in-line valve adapted to regulate IV fluid flow from full flow to an off position with definite variation between these positions with infinite variation between these positions by selective positioning of the variable area flow passageway 509.

The valve 502 is compact and is designed to be manually operated and in some applications its size and weight are an advantage as for example in an extension IV set where the light weight would exert little pull on the needle at the venapuncture site. However, in some other instances, it is desirable that the valve lend itself to one-hand operation. This allows the medical attendant to adjust the valve with one hand while performing other operations with the other hand. To this end, an optional backplate providing additional area for the user to grasp may be provided. The backplate of this embodiment is generally designated by the numeral 510 and can be molded as a onepiece unit from a suitable plastic material. The backplate includes a generally planar rear surface 512 and opposite forwardly extending side edges 514 and 516. The side edges are generally parallel and are spaced apart a distance less than the diameter of the body of valve 502. The side edges 514 and 516 both diverge outwardly at an intermediate location having a curvature generally corresponding to the curvature of the circular housing of the valve member 502 to form a receptacle 515 for housing 505 of the metering valve. In this way, valve 502 can be positioned in the backplate 510 and secured by an interference-fit with the valve body. Projections 525 extend in the receptacle 515 to engage the body 505 of the valve when inserted into the backplate. Note that in the assembled position, the diameter of the dial 504 extends the width of the backplate so that the operator can conveniently grasp the unit with the backplate resting in the palm of the hand and use the thumb and forefinger to grasp the dial from the rear to make the necessary adjustment.

The opposite ends of the backplate are recessed at 502 and 522 to accomodate the incoming and outgoing IV fluid lines 521 and 523. An upwardly extending projection 530 is provided at the upper left hand edge of the backplate as viewed in Figures 1 and 2. Projection 530 serves as part of a release mechanism when the valve is used with a cover as explained below. The lower opposite sides 514 and 516 are provided with apertures 532 and 534 to accomodate a cover if desired.

The cover which may be optionally attached to the backplate is generally designated by the numeral 550. The cover may be oppositely attached when it is desired to provide additional security to prevent either international or inadvertent adjustment of the setting of the valve as, for example, in the case of juvenile patients. The cover 500 includes a generally flat cover plate 552 having opposite depending sides 554 and 556. A portion of the cover is configured as a generally cylindrical receptacle adapted to receive the dial portion 504 of contained valve 502. The lower end of opposite sides 554 and 556 depend below the cover plate 552 and are provided with inwardly extending pins or axles 562 which are receivable within holes 532 and 534 at the opposite sides of the backplate. Since the device is preferably made of a suitable flexible plastic, sides 554 and 556 can be deflected outwardly a sufficient distance to engage the cooperating holes 532 and 534. Thus, when assembled, the cover is pivotally attached to the backplate and may be positioned in an open position as shown in Figure 6 or pivoted upwardly to a closed position as shown in Figure 1. Latch members 558 and 560 at the interior of the sides 554 and 556, engage the edge of the back cover to secure the cover in a closed position. Projection 575 extends upwardly from the upper right hand edge of the front cover as seen in Figure 6. Thus, to open the cover, projection 530 on the backplate and projection 575 on the cover plate may be easily forced in opposite directions to unlock the cover. A cut-out 580 is provided in the face of the cover so that the attendant can visually determine the setting of the valve. Alternatively, the backplate and cover may be molded as one piece and incorporate a connecting "live" hinge of a thin plastic membrane that allows opening and closing of the security cover.

In Figures 7 and 8, a preferred configuration for the valve is illustrated. Here the valve body 602 is generally cylindrical defining a valving chamber 604 having an inlet and an outlet 606 and 608, respectively. Annular flange 610 extends about valve body to receive rotary flow control member 615. The rotary flow control member includes a barrel 618 having a variable area metering slot 620 formed in the wall which is rotatable to regulate flow. The barrel 618 is preferably of a softer plastic material than housing body 602 for better sealing and is further selected to resist shrinkage. Barrel or body 618 has a concentric bore 626 with keyway 628 to receive axle 630 of the separate dial assembly 632. The dial 632 is of a more rigid plastic material and is locked in the barrel against rotation by an axial key 635 which is received in keyway 628. Annular locking member 640 with flanged section 642 engaged flange 610 to secure the assembly. The circular dial portion may have a beveled and serrated edge for better manual control.

Flange 610 has a vertically extending stop portion 650 which terminates at pointer 655. The rotation of the dial and associated barrel is limited as flange 642 will engage the stop 650. Thus, unidirectional operation can be achieved with increasing flow in one direction and decreasing in the other. Appropriate indicia may be placed on the face of the dial 632 which is cooperable with pointer 655 to provide an indication of flow setting.

In Figures 9 and 10 another embodiment of the present invention is shown which again includes a valve housing 102 having an inlet 104 and outlet 106 communicating with a flow chamber 108 defined by generally cylindrical housing 102. Again, the housing is fabricated preferably by injection molding from a selected plastic that is relatively hard as has been described above.

As seen in Figure 9, the rear surface 142 of the valve body 102 is provided with a pair of diametrically opposed lugs 145 which extend outward from the surface 142. Each of the lugs 145 has a radially extending wing or projection 147. Lugs 145 are integrally formed with the valve housing.

In the event the user requires additional gripping surface for holding the valve body when making adjustments to the flow rate, the optional backplate 150 as shown in Figure 10 may be attached to the rear of the valve body. The backplate 150 consists of an elongate panel 152 having a width generally corresponding to the width of the body of valve 102 and having sufficient length to extend at least to or beyond the termination of the inlet and outlet ports 104 and 106. The opposite sides of body 150 are provided with forwardly extending flanges 156 and 158 which provide a further gripping surface to facilitate manipulation of the valve. A pair of bayonet slots 160 and 162 are defined in the opposite sides of the valve body adapted to receive lugs 145. To secure the backplate 150, lugs 145 are inserted in the wide portion of the bayonet slots and the valve body is then rotated, locking the valve body to the valve plate. Appertures 168 and 169 may be provided at opposite ends of the backplate so the backplate and the valve can be secured to appropriate locations such as an IV stand.

Thus, it will be seen from the foregoing that the basic valve as described herein can be economically manufactured and provide the accuracy and repeatability necessary. In addition, the valve can further be provided with a backplate as an accessory to enhance the operation of the device and to make it compatible with one-hand operation. If security is desired, the optional cover plate can be attached to the backplate. Thus, in its simplest and economical form, the valve alone can be used in IV administration. For other applications, the medical attendant can add either the backplate or the backplate and the cover plate as required and dictated by the particular application and procedure.

The unique design and construction of the valving components avoids complex sealing arrangement and are economical to fabricate so the unit can be provided to health care facilities as an inexpensive, disposable item. This unit resists tampering and is accurate and simple for the medical attendants to operate giving accurate, repeatable results.

**Claims**

1. A valve (600) for establishing and maintaining predetermined administration flow rates in an intravenous system having a source of fluid connectable to an administration needle via tubing, said valve (600) comprising:
   (a) a valve housing (602) defining a valve chamber (604), and including an inlet (606) and an outlet (608) connectable to said tubing and placing said valve chamber (604) in the fluid flow path;
   (b) a flow control member (615) rotatable relative to said valve housing (602);
   (c) said valve housing (602) and flow control member (615) defining a flow passageway (620) being at least partially defined by a groove having a first region in which the cross-sectional area varies over its length, and a second flow-blocking region;
   (d) means (632) for selectively moving said flow control member (615) relative to said valve housing (602) to interpose a selected portion of said flow passageway (620) in said fluid path to vary the flow rate or block the fluid flow;

characterized in that:

(e) said valve chamber (604) is cylindrical and said flow control member (615) includes a barrel (618) having a cylindrical outer surface, said flow passageway (620) being at least partially defined by said groove on this cylindrical surface and having a third region of substantially uniform area for establishing a purge flow condition;

(f) said means (632) for selectively moving said flow control member (615) relative to said valve housing (602) including a separate dial assembly (632) provided with a cylindrical axle (630) and an axial key (635);

(g) said barrel (618) having a concentric bore (626) and an axial keyway (628) in which are received the axle (630) and the axial key (635) of said dial assembly (632);

(h) said valve housing (602) and said dial assembly (632) being made of a first sterilizable plastic material which will not flow or deform under hoop stress, the barrel (618) being made of a second plastic material selected to minimize heat shrinkage, the hardness of the second plastic material being lower than the hardness of the first plastic material as represented by Shore hardness to provide sealing and wiping action;

(i) the barrel (618) and the valve housing (602) being engaged in an interference fit, with the barrel (618) slightly compressed due to the relative dimensions of the barrel (618) and the valve housing (602).

2. A valve according to claim 1, wherein said housing is provided with stop means cooperable with said flow control member (504, 615) to limit movement thereof.

3. A valve according to any one of the preceding claims further including a backplate (150, 510) detachably secured to said housing.

4. A valve according to claim 3, wherein said backplate (150, 510) defines a recess (515) for receiving said valve housing and has a planar body (512) extending from said recess to provide a gripping surface.

5. A valve according to any one of claims 3 to 4, further including a cover face plate (550) which is movably securable to said backplate (150, 510) and valve (502) and which has an open position exposing said valve and a closed position covering said valve.

6. A valve according to claim 5, wherein said cover means (550) are securable to said backplate (150, 510) by pivotal means (562).

7. A valve according to any one of claims 5 and 6, wherein said cover plate is provided with a window (580) therein exposing a part of the dial (504) in said closed position.

8. A valve according to any one of the preceding claims, wherein said first plastic material is selected from the group consisting of ABS, XT and said second plastic material is selected from the group consisting of Neoprene, silicone, LDP or PVC.

9. A valve according to any one of the preceding claims, wherein said flow control member (615) is provided with flange means (642) which engage a portion of said valve housing.

**Patentansprüche**

1. Ventil (600) für die Festlegung und Aufrechterhaltung vorbestimmter darzureichender Durchflußmengen in einem intravenösen System mit einer Flüssigkeitsquelle, die über eine Leitung mit einer Einführnadel verbindbar ist, wobei

(a) das Ventil (602) ein als Ventilkammer (604) ausgebildetes und für dessen Anordnung in dem Flüssigkeitsweg mit der Leitung verbindbarem Einlaß (606) und Auslaß (608) versehenes Ventilgehäuse (602) und

(b) ein gegenüber dem Ventilgehäuse (602) verdrehbares Durchflüßregelelement (615) aufweist,

(c) das Ventilgehäuse (602) und das Durchflußregelelement (615) miteinander einen Durchflußweg (620) bilden, der zumindest teilweise als eine Nut mit einem ersten über seine Länge mit sich verändernden Querschnitt versehenen und mit einem zweiten den Durchflüß sperrenden Bereich ausgebildet ist und

(d) eine Einrichtung (632) für die selektive Verstellung des Durchflußregelelementes (615) gegenüber dem Ventilgehäuse (602) zum Zwischenschalten eines ausgewählten Teils des Durchflußweges (620) in den Flüssigkeitspfad zwecks Veränderung der Durchflußmenge oder Sperrung des Flüssigkeitsdurchgangs, dadurch gekennzeichnet, daß

(e) die Ventilkammer (604) zylindrisch ausgebildet ist und das Durchflußregelelement (615) eine Buchse (618) mit einer zylinderischen äußeren Oberfläche aufweist sowie die wenigstens teilweise den Durchflüßweg (620) bildende Nut in dieser zylindrischen Oberfläche ausgebildet ist und einen dritten Bereich mit im wesentlichen gleichbleibender Querschnittsfläche für die Erzielung der Durchflußbedingung für eine Reinigung aufweist,

(f) die Einrichtung (632) für die selektive Verstellung des Durchflußregelelementes (615) gegenüber dem Ventilgehäuse (602) eine separate Anzeigevorrichtung (632) mit einer zylindrischen Achse (630) und einem axialen Keil (635) aufweist,

(g) die Buchse (618) eine konzentrische Bohrung (626) und eine axiale Keilnut (628) für die Aufnahme der Achse (630) und des axialen Keils (635) der Anzeigevorrichtung (632) aufweist,

(h) das Ventilgehäuse (602) und die Anzeigevorrichtung (632) aus einem ersten sterilisierbaren Plastikmaterial hergestellt sind, welches unter Ringspannung nicht fließt oder sich deformiert und die Buchse (618) zwecks Erzielung einer Dichtungs- und Wischwirkung aus einem zweiten Plastikmaterial hergestellt ist, das im Sinne der Minimalisierung der Hitze-Schrumpfung ausgewählt ist und dessen Shore-Härte geringer ist als die Shore-Härte des ersten Plastikmaterials,

(i) die Buchse (618) und das Ventilgehäuse (602) miteinander mittels einer Passung mit Übermaß in Eingriff stehen, wobei durch entsprechende Abmessungen der Buchse (618) und des Ventilgehäuses (602) die Buchse (618) schwach zusammengepreßt ist.

2. Ventil nach Anspruch 1, dadurch gekennzeichnet, daß das Gehäuse mit Anschlagelementen versehen ist, die mit dem Durchflußregelelement (504, 615) zusammenarbeiten und dessen Bewegung begrenzen.

3. Ventil nach Anspruch 1 oder 2, dadurch gekennzeichnet, daß es eine mit dem Gehäuse lösbar verbundene Rückwand (150, 510) aufweist.

4. Venteil nach Anspruch 3, dadurch gekennzeichnet, daß die Rückwand (150, 510) eine Vertiefung (515) für die Aufnahme des Ventilgehäuses und einen sich von der Vertiefung aus erstreckenden ebenen Boden (512) zur Erzielung einer Einspannfläche aufweist.

5. Ventil nach Anspruch 3 oder 4, dadurch gekennzeichnet, daß es eine vordere Deckplatte (550) aufweist, die beweglich mit der Rückwand (150, 510) und dem Ventil (502) verbindbar ist und die in Offenstellung das Ventil freigibt und in Verschlußstellung das Ventil abdeckt.

6. Ventil nach Anspruch 5, dadurch gekennzeichnet, daß die vordere Deckplatte (550) mit der Rückwand (150, 510) mittels Führungszapfen (562) verbindbar ist.

7. Ventil nach Anspruch 5 oder 6, dadurch gekennzeichnet, daß die vordere Deckplatte mit einem Fenster (580) versehen ist, das in Verschlußstellung einen Teil der Anzeige (504) freigibt.

8. Ventil nach einem der vorhergehenden Ansprüche, dadurch gekennzeichnet, daß das erste Plastikmaterial aus der Gruppe bestehend aus ABS, XT und das zweite Plastikmaterial aus der Gruppe bestehend aus Neopren, Silicon, LDP oder PVC ausgewählt ist.

9. Ventil nach einem der vorhergehenden Ansprüche, dadurch gekennzeichnet, daß das Durchflußregelelement (615) mit Flanschelementen (642) versehen ist, die mit einem Teil des Ventilgehäuses in Eingriff bringbar sind.

## Revendications

1. Valve (600) pour établir et maintenir des débits d'administration prédéterminés dans un système intraveineux comportant une source de fluide pouvant être raccordée à une aiguille d'administration par l'intermédiaire d'un tuyau, la valve (600) comprenant:

(a) un corps de valve (602) délimitant une chambre de valve (604) et comprenant une entrée (606) et une sortie (608) pouvant être raccordée au tuyau et plaçant la chambre de valve (604) dans le trajet d'écoulement de fluide;

(b) un organe de reglage d'écoulement (615) pouvant tourner par rapport au corps de valve (602);

(c) le corps de valve (602) et l'organe de réglage d'écoulement (615) définissant un passage d'écoulement (620) qui est formé au moins partiellement par une gorge comportant une première région, dont l'aire en coupe varie dans le sens de sa longueur, et une seconde région de blocage de l'écoulement;

(d) un moyen (632) pour déplacer sélectivement l'organe de réglage de l'écoulement (651) par rapport au corps de valve (602) afin d'interposer une partie sélectionnée du passage d'écoulement (620) dans le trajet de fluide pour faire varier le débit de l'écoulement de fluide ou bloquer cet écoulement,

caractérisé en ce que:

(e) la chambre de valve (604) est cylindrique et l'organe de réglage d'écoulement (615) comprend un barillet (618) comportant une surface extérieure cylindrique, le passage d'écoulement (620) étant défini au moins partiellement par la gorge dans cette surface cylindrique et comportant une troisième région de section en substance uniforme pour établir un état d'écoulement de purge;

(f) le moyen (632) destiné à déplacer sélectivement l'organe de réglage d'écoulement (615) par rapport au corps de valve (602) comprend un élément à cadran séparé (632) pourvu d'un axe cylindrique (630) et d'une clavette axiale (635);

(g) le barillet (618) présentant un alésage concentrique (626) et une rainure de clavette axiale (628) qui reçoivent l'axe (630) et la clavette axiale (635) de l'élément à cadran (632);

(h) le corps de valve (602) et l'élément à cadran (632) étant faits d'une première matière plastique stérilisable qui ne subit aucun fluage ni déformation sous l'effet d'une contrainte circonférentielle, le barillet (618) étant fait d'une seconde matière plastique choisie afin de réduire au minimum tout retrait thermique, la dureté de cette seconde matière plastique étant inférieure à celle de la première, sur une échelle de dureté Shore, afin d'assurer un effet d'étanchéité et de frottement, et

(i) le barillet (618) et le corps de valve (602) étant engagés avec serrage, le barillet (618) étant légèrement comprimé en raison de ses dimensions propres de celles du corps de valve (602).

2. Valve suivant la revendication 1, dans laquelle le corps est pourvu de moyens d'arrêt pouvant coopérer avec l'élément de réglage d'écoulement (504, 615) pour limiter le déplacement de ce dernier.

3. Valve suivant l'une quelconque des revendications précédentes comprenant, en outre, une plaque dorsale (150, 510) fixée de manière détachable au corps.

4. Valve suivant la revendication 3, dans laquelle la plaque dorsale (150, 510) définit un logement (515) destiné à recevoir le corps de valve et présente un corps plan (512) qui s'étend à partir du logement pour former une surface d'agrippage.

5. Valve suivant l'une quelconque des revendications 3 et 4 comprenant, en outre, une plaque frontale formant couvercle (550) qui peut être fixée de manière mobile à la plaque dorsale (150, 510) et à la valve (502) et qui présente une position ouverte exposant la valve et une position fermée couvrant cette valve.

6. Valve suivant la revendication 5, dans laquelle le couvercle (550) peut être fixé à la plaque dorsale (150, 510) par des moyens de pivotement (562).

7. Valve suivant l'une quelconque des revendications 5 et 6, dans laquelle le couvercle est pourvu d'une fenêtre (580) exposant une partie du cadran (504) dans sa position de fermeture.

8. Valve suivant l'une quelconque des revendications précédentes, dans laquelle la première matière plastique est choisie dans le groupe comprenant l'ABS, le XT et la seconde matière plastique est choisie dans le groupe comprenant le Néoprène, le silicone, le LDP et le PVC.

9. Valve suivant l'une quelconque des revendications précédentes, dans laquelle l'organe de réglage d'écoulement (615) est pourvu de languettes (642) qui sont en prise avec une partie du corps de valve.

FIG-2

FIG-1

FIG-3

FIG-4

FIG-5

1

EP 0 187 827 B1

FIG-6

FIG-8

FIG-7

2

_Fig - 9_

_Fig - 10_